(19) Europäisches Patentamt
European Patent Office
Office européen des brevets

(11) Publication number: 0 036 030
B1

(12) EUROPEAN PATENT SPECIFICATION

(45) Date of publication of patent specification: 13.06.84

(51) Int. Cl.³: C 07 D 495/04
//A61K31/415, (C07D495/04, 333/00, 235/00)

(21) Application number: 80101360.8

(22) Date of filing: 14.03.80

(54) **Process for preparing biotin.**

(43) Date of publication of application:
23.09.81 Bulletin 81/38

(45) Publication of the grant of the patent:
13.06.84 Bulletin 84/24

(84) Designated Contracting States:
CH DE FR GB IT

(56) References cited:
US - A - 3 740 416

(73) Proprietor: SUMITOMO CHEMICAL COMPANY, LIMITED
15 Kitahama 5-chome Higashi-ku
Osaka-shi Osaka-fu (JP)

(72) Inventor: Takahashi, Takeo
3-8-10, Nakayamasatsukidai
Takarazuka, Hyogo (JP)
Inventor: Shimago, Kozo
2-10-4-412, Sonehigashimachi
Toyonaka, Osaka (JP)
Inventor: Maeshima, Kaoru
2-14-7, Mefu
Takarazuka, Hyogo (JP)

(74) Representative: Vossius Vossius Tauchner Heunemann Rauh
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 036 030 B1

**Description**

The present invention relates to a process for preparing biotin.

Biotin, the chemical name of which is cis-tetra-hydro-2-oxothieno[3,4-d]imidazoline-4-valeric acid, has an asymmetric carbon, and the term "biotin" in the present invention includes both of *d*-biotin and *dl*-biotin.

Biotin (also called "Vitamin H") is a valuable substance exerting a growth accelerative effect as well as a preventive and therapeutic effect on dermatoses, etc.

For the production of biotin, there is known a method wherein 3,4-(1′,3′-dibenzyl-2′-keto-imidazolido)-2-(ω-carboxybutyl)thiophane is heated under reflux in 48% hydrobromic acid to effect debenzylation (U.S. Patent No. 3,740,416). However, this method is not favourable because it gives the objective compound only in a poor yield in spite of the troublesome operation due to the use of an irritative reagent.

As the result of an extensive study, it has now been established an improved process for preparing biotin in an excellent yield with a high purity and without any disadvantages as seen in the said conventional process.

Thus, the present invention provides an industrially advantageous process for preparing biotin which comprises heating a compound of the formula:

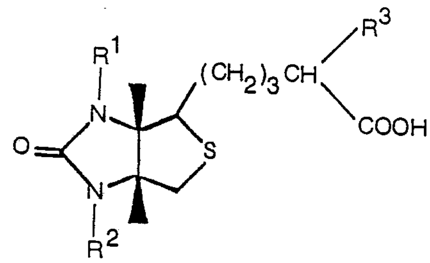

[I]

wherein $R^1$ and $R^2$ are each hydrogen or benzyl and $R^3$ is hydrogen or carboxyl, with the proviso that $R^1$ and $R^2$ are not simultaneously hydrogen in the presence of an alkanesulfonic acid containing 1 to 3 carbon atoms.

According to the invention, a mixture of one part by weight of the starting compound [I] and 1 to 100 parts by weight, preferably 3 to 50 parts by weight, of an alkanesulfonic acid containing 1 to 3 carbon atoms (e.g., methanesulfonic acid, ethanesulfonic acid, propanesulfonic acid) is heated at a temperature of 80 to 180°C, preferably of 110 to 150°C, for a period of 10 minutes to 20 hours, preferably of 0.5 to 10 hours. The reaction mixture is cooled and then admixed with water. Precipitated crystals are collected by filtration and recrystallized from water to give purified crystals of biotin.

It is not requisite but preferred to add to the reaction system a hydrocarbon having a boiling point higher than 100°C (e.g., chlorobenzene, toluene, xylene, octane, decane, anisole) and/or an alkanoic acid, preferably having 2 to 4 carbon atoms (e.g., acetic acid, propionic acid), in order to minimize the formation of by-products and easily control the reaction temperature.

The starting compound [I] may be derived from 3,4-(1′,3′-dibenzyl-2′-ketoimidazolido)-2-(ω,ω-di(lower)alkoxy-carbonylbutyl)thiophane (hereinafter referred to as "compound [II]") by saponification; optionally followed by decarboxylation and/or partial debenzylation. Specific examples of the compound [II] are 3,4-(1′,3′-dibenzyl-2′-ketoimidazolido)-2-(ω,ω-dimethoxycarbonylbutyl)thiophane, 3,4-(1′,3′-dibenzyl-2′-ketoimidazolido)-2-(ω,ω-diethoxycarbonylbutyl)thiophane, 3,4-(1′,3′-dibenzyl-2′-ketoimidazolido)-2-(ω-methoxycarbonyl-ω-ethoxycarbonylbutyl)thiophane.

The starting compound [I] thus prepared may be separated from the reaction mixture and subjected to the process of the invention. However, it can be directly converted to biotin in situ, i.e., without separation from the reaction mixture. The typical example for preparing biotin from the compound [II] without separation of the compound [I] is illustrated below.

A mixture of the compound [II] and the alkane-sulfonic acid containing water (water content, usually not more than 40% by weight) is heated at a temperature of 40 to 100°C, preferably of 70 to 95°C, for 2 to 20 hours in the presence of absence of an alkanoic acid, preferably having 2 or 3 carbon atoms, whereby saponification proceeds to give a reaction mixture containing 3,4-(1′,3′-dibenzyl-2′-ketoimidazolido)-2-(ω,ω-dicarboxybutyl)thiophane. The reaction mixture is continuously heated at a temperature of 100 to 180°C, preferably of 110 to 150°C, for 1 to 20 hours while distilling off volatile components (e.g., water, alcohols, esters), whereby decarboxylation and debenzylation proceed to give a reaction mixture containing biotin. The reaction mixture is worked up in the same manner as previously mentioned to give purified crystals of biotin.

This modified process for preparing biotin from the compound [II] without separation of the compound [I] constitutes one embodiment of the present invention.

The compound [II] is obtainable by reacting 3,4-(1′,3′-dibenzyl-2′-ketoimidazolido)-1,2-tri-

methylenethiophanium bromide or *d*-camphor-sulfonate with a malonic di-ester according to U.S. patent 3,740,416 or 2,489,235.

As stated above, the process of this invention can advantageously afford biotin in an excellent yield with a high purity. It is particularly notable that biotin of high optical purity is obtainable by the use of the starting compound [I] which is optically active. Accordingly, this invention can provide an extremely important and valuable process for the synthesis of *d*-biotin which is, in particular, physiologically active.

Practical and presently preferred embodiments of the invention are illustratively shown in the following Examples.

Example 1

*d*-Monobenzylbiotin (1.0 g) and methanesulfonic acid (3.0 g) are introduced into a 10 ml volume flask, and the flask is heated in an oil bath kept at 130°C while vigorous stirring with a magnetic stirrer for 6 hours. The reaction mixture is cooled to room temperature and poured into 10 ml of ice water while stirring. Precipitated crystals are collected by filtration and recrystallized from water to give biotin (white needles, 0.58 g). Yield, 79%. M.P. 230°C. $[\alpha]_D^{20} = +91°$ (C = 1 in 0.1% NaOH aq.).

Reference Example 1

Diethyl malonate (45.0 g) is dropwise added to a suspension of sodium methoxide (11 g) in toluene (200 ml) kept at 80°C, and the mixture is stirred for one hour while keeping this temperature. The reaction mixture is cooled to 50°C, l-(-)-3,4-(1',3'-dibenzyl-2'-ketoimidazolido)-1,2-trimethylenethiophanium bromide (30.0 g) is added thereto and the resultant mixture is heated under reflux for 4 hours. The reaction mixture is cooled to room temperature and admixed with water (270 g). The organic layer is separated, washed with water (250 g), dried and distilled in vacuo to give the residue primarily containing l-(-)-3,4-(1',3'-dibenzyl-2'-ketoimidazolido)-2-(ω,ω-diethoxycarbonylbutyl)-thiophane. To the residue, there is added ethanol (160 g) containing 24.1% by weight of KOH, and the mixture is heated under reflux for one hour. By removing the solvent in vacuo, there is obtained the residue, which is admixed with water (300 g). The resulting mixture is neutralized with conc. hydrochloric acid and extracted with ethyl acetate. The organic layer is dried and distilled in vacuo to give the residue primarily containing l-(-)-3,4-(1',3'-dibenzyl-2'-ketoimidazolido-2-(ω,ω-dicarboxybutyl)-thiophene.

Example 2

Methanesulfonic acid (90.0 g) is added to the residue obtained in Reference Example 1, and the mixture is heated with stirring in an oil bath kept at 130°C for 5 hours. The reaction mixture is cooled to room temperature, and ice cold water (270 g) is dropwise added thereto to give a slurry. The slurry is filtered with suction, and the obtained crystals are recrystallized from water to give *d*-biotin (white needles, 12.6 g). Yield, 77% (based on l-(-)-3,4-(1',3'-dibenzyl-2'-ketoimidazolido)-1,2-triethylenethiophanium bromide). $[\alpha]_D^{20} = +90°$ (C = 1 in 0.1% NaOH aq.).

Example 3

Formic acid (75 g) and methanesulfonic acid (10.0 g) are added to the residue containing l-(-)-3,4-(1',3'-dibenzyl-2'-ketoimidazolido)-2-(ω,ω-diethoxycarbonylbutyl)-thiophane, obtained in the same manner as in Reference Example 1, and the mixture is heated with stirring at 90°C for 4 hours. By raising the temperature up to about 150°C, the low boiling point components are distilled off. The mixture is cooled to 100°C again, admixed with additional methanesulfonic acid (150 g), and the mixture is heated with stirring at 140°C for 4 hours. The reaction mixture is cooled and poured into ice water (500 g) to obtain a slurry. The slurry is treated in the same manner is in Example 2 to give *d*-biotin (12.3 g). Yield, 75.1% (based on l-(-)-3,4-(1',3'-dibenzyl-2'-ketoimidazolido)-1,2-trimethylenethiophanium bromide).

Example 4

Methane sulfonic acid (200.0 g) and crude N,N-dibenzylbiotin (20.0 g) are introduced into a four-necked flask, and the mixture is stirred at 140°C for 6 hours. The reaction mixture is cooled to a temperature between 10 and 15°C and poured into ice water (1 l) while stirring. the precipitated crystals are collected by filtration and recrystallized from water to give *d*-biotin (white needles, 8.30 g). Yield, 72.0%.

Example 5

Methanesulfonic acid (120 g) and acetic acid containing water (water content, about 40%) (50 ml) are added to the residue containing *dl*-3,4-(1',3'-dibenzyl-2'-ketoimidazolido)-2-(ω,ω-diethoxycarbonylbutyl)thiopane, obtained in the same manner as in Reference Example 1 but using *dl*-3,4-(1',3'-dibenzyl-2'-ketoimidazolido)-1,2-trimethylenethiophanium *d*-camphor-sulfonate (39.9 g) in place of l-(-)-3,4-(1',3'-dibenzyl-2'-ketoimidazolido)-1,2-trimethylenethiophanium bromide, and the resulting mixture is heated with stirring at a temperature of 93 to 95°C for 6 hours. By raising the

temperature to about 140°C, the low boiling point components are removed. Xylene (100 ml) is added to the residue, and the mixture is heated under reflux for 8 hours, during which a refluxing liquid predominantly containing xylene (about 30 ml) is removed from the reaction system. The reaction mixture is cooled to room temperature, the xylene layer is separated off and the remaining layer is poured into ice water (400 ml). The precipitated crystals are collected by filtration and recrystallized from water to give *dl*-biotin (white needles, 11.5 g). M.P. 233 to 235°C.

**Claims**

1. A process for preparing biotin, characterized in that one part by weight of a compound of the formula:

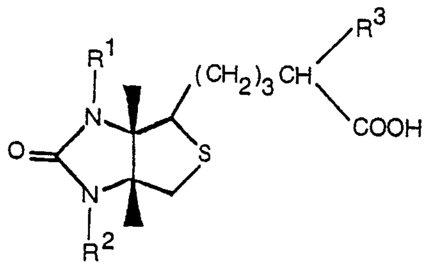

[I]

wherein $R^1$ and $R^2$ are each hydrogen or benzyl and $R^3$ is hydrogen or carboxyl, with the proviso that $R^1$ and $R^2$ are not simultaneously hydrogen, is heated at 80 to 180°C in the presence of 1 to 100 parts by weight of an alkanesulfonic acid containing 1 to 3 carbon atoms for a period of 10 minutes to 20 hours.

2. The process according to claim 1, wherein $R^1$ or $R^2$ is benzyl.

3. The process according to claim 1 or 2, wherein $R^3$ is hydrogen.

4. The process according to any one of claims 1 to 3, wherein the alkanesulfonic acid is methanesulfonic acid.

5. The process according to any one of claims 1 to 4, wherein the reaction is effected in the presence of a hydrocarbon having a boiling point higher than 100°C and/or an alkanoic acid having 2 to 4 carbon atoms.

6. The process according to claims 1 to 5, wherein the reaction is effected at a temperature of 110 to 150°C.

**Revendications**

1. Procédé de préparation de biotine, caractérisé en ce qu'une partie en poids d'un composé de formule:

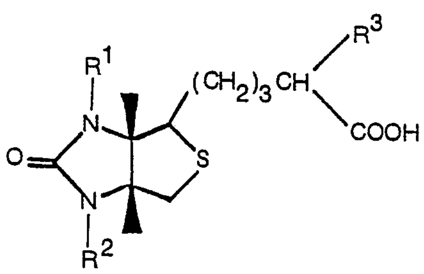

[I]

où $R^1$ et $R^2$ représentent chacun l'hydrogène ou le groupe benzyle et $R^3$ est l'hydrogène ou le groupe carboxyle, en prévoyant que $R^1$ et $R^2$ ne sont pas simultanément l'hydrogène, est chauffée entre 80 et 180°C en présence de 1 à 100 parties en poids d'un acide alcanesulfonique contenant 1 à 3 atomes de carbone pendant une période de 10 minutes à 20 heures.

2. Procédé selon la revendication 1, dans lequel $R^1$ ou $R^2$ est le groupe benzyle.

3. Procédé selon la revendication 1 ou 2, dans lequel $R^3$ est l'hydrogène.

4. Procédé selon l'une quelconque des revendications 1 à 3, dans lequel l'acide alcanesulfonique est l'acide méthanesulfonique.

5. Procédé selon l'une quelconque des revendications 1 à 4, dans lequel la réaction est effectuée en présence d'un hydrocarbure ayant un point d'ébullition supérieur à 100°C et/ou d'un acide alcanoïque ayant 2 à 4 atomes de carbone.

6. Procédé selon l'une quelconque des revendications 1 à 5, dans lequel la réaction est efectuée à une température de 110 à 150°C.

## Patentansprüche

1. Verfahren zur Herstellung von biotin, dadurch gekennzeichnet, daß man 1 Gewichtsteil einer Verbindung der Formel:

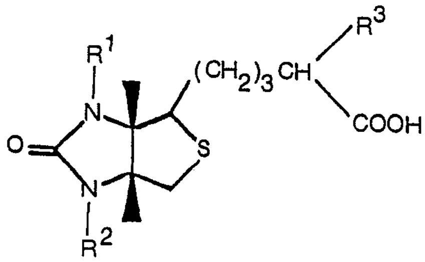

[I]

in der $R^1$ und $R^2$ jeweils ein Wasserstoffatom oder eine Benzylgruppe bedeuten und $R^3$ ein Wasserstoffatom oder eine Carboxylgruppe darstellt, mit der Maßgabe, daß $R^1$ und $R^2$ nicht gleichzeitig Wasserstoffatome darstellen, in Gegenwart von 1 bis 100 Gewichtsteilen einer Alkansulfonsäre mit 1 bis 3 Kohlenstoffatomen über einen Zeitraum von 10 Minuten bis 20 Stunden auf 80°C bis 180°C erhitzt.

2. Verfahren nach Anspruch 1 dadurch gekennzeichnet, daß $R^1$ oder $R^2$ eine Benzylgruppe bedeuten.

3. Verfahren nach Anspruch 1 oder 2, dadurch gekennzeichnet, daß $R^3$ ein Wasserstoffatom bedeutet.

4. Verfahren nach jedem der Ansprüche 1 bis 3, dadurch gekennzeichnet, daß man als Alkansulfonsäure die Methansulfonsäure verwendet.

5. Verfahren nach jedem der Ansprüche 1 bis 4, dadurch gekennzeichnet, daß man die Umsetzung in Gegenwart eines Kohlenwasserstoffs, der einen Siedepunkt von höhler als 100°C hat und/oder einer Alkansäure mit 2 bis 4 Kohlenstoffatomen durchführt.

6. Verfahren nach den Ansprüchen 1 bis 5, dadurch gekennzeichnet, daß man die Umsetzung bei Temperaturen von 110°C bis 150°C durchführt.